(19) **Europäisches Patentamt / European Patent Office / Office européen des brevets**

(11) **EP 3 417 401 B1**

(12) **EUROPEAN PATENT SPECIFICATION**

(45) Date of publication and mention
of the grant of the patent:
**19.01.2022 Bulletin 2022/03**

(21) Application number: **17754001.0**

(22) Date of filing: **17.02.2017**

(51) International Patent Classification (IPC):
*A61B 5/00* (2006.01)  *A61B 5/02* (2006.01)

(52) Cooperative Patent Classification (CPC):
**A61B 5/7203; A61B 3/102; A61B 3/12;
A61B 5/0066; A61B 5/02007; A61B 5/7207;
A61B 5/7253; A61B 5/7267; G06K 9/0051;
G06K 9/40; G06T 5/001;** A61B 5/0261;
G06T 2207/10101

(86) International application number:
**PCT/US2017/018521**

(87) International publication number:
**WO 2017/143300 (24.08.2017 Gazette 2017/34)**

(54) **METHOD FOR REDUCING ARTIFACTS IN OCT USING MACHINE LEARNING TECHNIQUES**

VERFAHREN ZUR VERRINGERUNG VON ARTEFAKTEN IN OCT MITHILFE VON TECHNIKEN ZUM MASCHINELLEN LERNEN

PROCÉDÉ DE RÉDUCTION D'ARTEFACTS DANS UNE OCT GRÂCE À DES TECHNIQUES D'APPRENTISSAGE AUTOMATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.02.2016 US 201662297649 P**

(43) Date of publication of application:
**26.12.2018 Bulletin 2018/52**

(73) Proprietor: **Optovue, Inc.**
**Fremont, CA 94538 (US)**

(72) Inventors:
• **HSIAO, Yi-Sing**
**Union City**
**CA 94587 (US)**
• **JANG, Ben K.**
**Cupertino**
**CA 95014 (US)**

• **SHARMA, Utkarsh**
**Dublin**
**CA 94558 (US)**
• **ZHOU, Qienyuan**
**San Diego, CA 92130 (US)**
• **KO, Tony H.**
**Cupertino**
**CA 95014 (US)**
• **WEI, Jay**
**Fremont**
**California 94539 (US)**

(74) Representative: **Gill, David Alan**
**WP Thompson**
**138 Fetter Lane**
**London EC4A 1BT (GB)**

(56) References cited:
**US-A1- 2011 267 340    US-A1- 2015 062 590
US-A1- 2015 110 348    US-A1- 2015 110 348
US-A1- 2016 040 977**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**RELATED APPLICATION**

The present disclosure claims priority to U.S. Patent Application No. 15/436,704, filed on February 17, 2017, now U.S. Patent No. 10,194,866, which claims the benefit of U.S. Provisional Application No. 62,297,649, filed on February 19, 2016.

**BACKGROUND**

*Technical Field*

Embodiments of the present invention relate generally to the field of optical coherence tomography (OCT) angiography and applications thereof, and specifically methods and apparatus for improved processing of OCT angiography (OCTA) data and reducing the effects of various errors caused by, for example, projection artifacts, low OCT signal, and noise.

*Discussion of Related Art*

Optical coherence tomography angiography (OCTA) is a non-invasive vascular imaging modality to visualize flow by detecting motion contrast using repeated OCT measurements at the same location. *See, e.g.,* Talisa E. de Carlo et al. "A review of optical coherence tomography angiography (OCTA)," Int J Ret Vit, April 2015. Unlike fluorescein angiography (FA) and indocyanine green (ICG) angiography, OCTA imaging is injection-free and provides depth-resolved three dimensional (3D) vascular information of the blood flow or vasculature in the tissue such as in an eye. While FA still remains the gold standard for diagnosis of ocular pathologies that result in abnormal vascular function, OCTA is a highly promising technique that may provide similar or, at times, complementary information regarding the vasculature abnormality, without the invasiveness associated with FA.

However, the clinical utility of OCTA can be greatly impacted by various sources of error that could lead to imaging artifacts and erroneous analysis. See, e.g., Richard F. Spaide et al. "Image Artifacts in Optical Coherence Tomography Angiography," Retina, Nov 2015. These artifacts include, but are not limited to, projection artifacts, shadowing caused by low OCT signal, and noise.

US 2015/062590 A1 discloses an efficient method of evaluating the level of contrast of an OCT dataset is presented and which develops a metric to segregate useful and not-so-useful data in one or more OCT B-scans, in order to reduce spurious subsequent analyses of the data by downstream segmentation algorithms.

US 2015/110348 A1 discloses systems and methods that aid in screening, diagnosis and/or monitoring of medical conditions and whcih may allow, for example, for automated identification and localization of lesions and other anatomical structures from medical data obtained from medical imaging devices, computation of image-based biomarkers including quantification of dynamics of lesions, and/or integration with telemedicine services, programs, or software.

Therefore, methods and apparatus to mitigate for errors to increase the clinical utility of OCTA is needed.

**SUMMARY**

The invention is defined by the appended claims.

**BRIEF DESCRIPTION OF THE DRAWINGS**

Figure 1 illustrates an exemplary image of the OCTA B-scan with projection artifacts.

Figures 2A through 2D illustrate exemplary images of OCTA imaging of a normal subj ect.

Figure 3 shows a block diagram illustrating the steps to reduce projection artifacts in OCTA 3D volume.

Figure 4 illustrates an exemplary image of the OCTA B-scan at the same location as Fig. 1 after projection artifacts are reduced.

Figures 5A through 5D illustrate exemplary images of OCTA imaging of the same normal subject shown in Fig. 2 after the projection artifacts are reduced.

Figures 6A and 6B illustrate exemplary images of the outer retina of an age-related macular degeneration (AMD) patient with choroidal neovascularization (CNV) before and after projection artifacts are reduced in the OCTA volume.

Figure 7 shows a block diagram illustrating the steps to train the classifier used for projection artifacts reduction.

## DETAILED DESCRIPTION

In the following description, specific details are set forth describing some embodiments of the present invention. The invention is defined by the appended claims.

OCTA imaging detects vessels with blood flow. The terms "flow" and "vessel" are therefore used interchangeably in the following descriptions. OCTA employs motion contrast imaging in order to generate images that show flow, in particular blood flow. In particular, an OCTA imager compares the differences in the backscattered OCT signal intensity between sequential OCT B-scans taken at the same cross-section of the sample in order to construct a map of blood flow. As has been discussed elsewhere, the OCT scans can be corrected for eye movement between sequential images. In some systems, both the OCT images and the derivative OCTA images can be provided.

Projection or decorrelation-tail artifacts are one of the most important artifacts that could limit the clinical utility and accuracy of OCTA results. Current OCTA processing techniques can generate false motion contrast signals in tissue that falls underneath a blood flow region, even when the underlying tissue is static. OCTA techniques are based on the principle of obtaining motion contrast, i.e. identifying and quantifying the change in OCT signal at different depths in the tissue. When the light passes through a blood vessel or a flow region, various factors such as forward scattering, refraction, absorption and path length variations cause unpredictable changes to the light field (and signal) at subsequent depths. The backscattered light (and hence the signal) that comes from underneath a region of flow inherits the changes in light field and signal from above, and hence may show a false motion contrast signal, depending on the level of backscattered light and change imparted by the disturbance above. It is very difficult to quantify or correct these changes as they are variable in nature and change in each measurement.

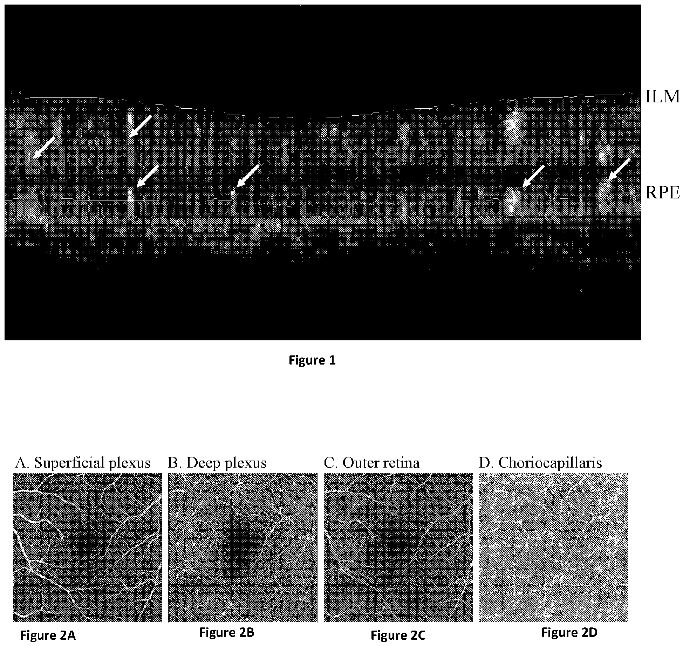

Figure 1 illustrates an exemplary image of the OCTA B-scan illustrating projection artifacts. In Figure 1, some projection artifacts are illustrated by arrows. Figure 1 further shows the internal limiting membrane (ILM) and retinal pigment epithelial (RPE). Figure 1 shows OCTA signal in the human retinal layers. The arrows indicate the projection artifacts at different retinal levels, whereas the true location of the blood vessels is in the retina above. Hence, any quantitative analysis that occurs without removing the projection artifacts will be misleading, inaccurate, or sub-optimal at best.

Previous methods to reduce projection artifacts have been disclosed and are based on two dimensional (2D) image processing. *See, e.g.,* Yali Jia et al. "Quantitative Optical Coherence Tomography Angiography of Choroidal Neovascularization in Age-Related Macular Degeneration," Ophthalmology, July 2014; and Anqi Zhang et al. "Minimizing Projection Artifacts for Accurate Presentation of Choroidal Neovascularization in OCT Micro-Angiography," Biomed Opt Exp, Sep 2015. In these methods, retinal layer segmentation is required before reducing the projection artifacts, which poses a big limitation because segmentation in pathological tissues may be inaccurate. In addition, 3D visualization and analysis are still not feasible with these approaches. A method was proposed to reduce the projection artifacts in a 3D manner (Miao Zhang et al. "Projection-resolved optical coherence tomographic angiography," Biomed Opt Exp, Mar 2016), however, it uses a simple observational criterion and removes suspicious artifacts completely, causing vascular breakage and large shadowing in both inner retina and choroid layers. Therefore, methods and apparatus to reduce artifacts in the OCTA volume, specifically in a 3D manner while maintaining the intactness of vascular networks, are needed for better visualization and quantitative measurements.

Shadowing artifacts occur when the OCT signal is attenuated behind an absorbing of scattering opacity or obstruction. No or low OCT signal results in no or low OCTA signal. These artifacts can be due to the pathologies of patients such as epi-retinal membranes (floaters) and cataracts. The artifacts can also be due to strong light absorption in the upper tissue layers. Some imaging and processing techniques may be applied to alleviate the shadowing effect. Subsequent image processing and analysis for OCTA can be adjusted accordingly to offset the shadowing effect.

Another artifact is noise. System noise and fluctuations in OCT incident light intensity can result in high OCTA signal even at locations of static tissue with no flow. OCTA noise, or false-positive flow, can be visually identified by its short segment and isolation from neighboring structured vessels. However, the presence of noise affects subsequent quantification and visualization of small capillaries.

Overall, these artifacts or different combinations of these can significantly degrade the clinical utility of OCTA results and lead to erroneous conclusions. Some embodiments of the present invention provide solutions to mitigate these challenges and reduce the number of artifacts in the resulting OCTA images.

OCTA volume data may consist of artificial signals that are not related to flow. The errors in the OCTA data caused by factors including projection artifacts, shadowing, and noise can be detected and reduced through several methods and techniques according to some embodiments and discussed in this disclosure. This reduction in the artifacts can result in improving the image quality of retinal microvasculature visualization and accuracy of the subsequent quantitative measurements for blood flow.

The methods used to reduce OCTA artifacts can be generalized to process both OCT and OCTA 3D volume, 2D plane (B-scan), and 1D line (A-line) data. After applying one or more processing methods to reduce artifacts in the OCTA 3D volume, the artifacts-reduced volume can be used for true 3D visualization. In other embodiments, the artifacts-reduced volume can be used to generate 2D en face projection images. The methods to generate en face images have been disclosed in previous applications. *See, e.g.,* John Davis et al. "Enhanced imaging for optical coherence tomography," US Patent US 8,781,214 B2, July 2014.

In some embodiments, the OCTA data can be visualized in 3D and/or 2D by using a different color scheme for pre-processed original signals and artifact signals. For example, voxels/pixels with true signals can be color-coded in grayscale, while projection artifacts color-coded in red, and shadowing artifacts in blue.

Furthermore, vascular parameters can be calculated from the artifacts-reduced OCTA volume. In some embodiments, quantitative measurements can be calculated with 3D volume-based parameters and/or 2D en face image-based parameters. The parameters include, but are not limited to, flow volume/area, non-flow volume/area, flow density (volume/area/length density), vessel caliber, vessel branching, and tortuosity.

### *Projection Artifacts*

Figures 1 and 2A through 2D illustrate projection artifacts in a normal subject with no retinal pathologies based on clinical evaluation, as demonstrated by the B-scan (Fig. 1) and en face (Figs. 2A through 2D) images. Figures 2A through 2D illustrate exemplary images of OCTA imaging of a normal subject, with Figure 2A illustrating en face images of four retinal layers, superficial capillary plexus, with Figure 2B illustrating the deep capillary plexus, with Figure 2C illustrating the outer retina, and with Figure 2D illustrating the choriocapillaris. Figures 2A through 2D have been generated from the pre-processed OCTA volume before projection artifacts are reduced.

The projection artifacts appear at different retinal layers, as indicated by the arrows in Figure 1. The projection artifacts coming from the superficial capillary plexus (Figure 2A) are most noticeable, causing false OCTA signals with similar vascular pattern in the deep capillary plexus (Figure 2B), outer retina (Figure 2C), and choriocapillaris (Figure 2D) layers, where no capillaries actually exist.

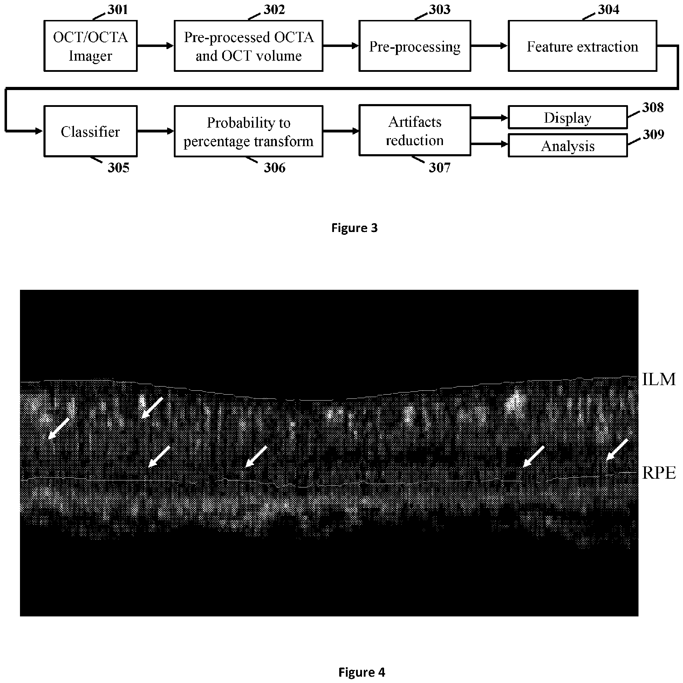

Figure 3 illustrates an exemplary flow diagram demonstrating the steps to reduce artifacts in an OCTA 3D volume. An OCTA imager (block 301) generates OCTA volume from OCT data using methods described in previously filed applications. *See, e.g.,* Yali Jia et al. "Split-spectrum amplitude-decorrelation angiography with optical coherence tomography," Optics Express, Feb 2012. In another embodiment, an OCT imager can also be used to provide the structural OCT volume for additional information. The OCTA and OCT imager can also be combined to a single OCT/OCTA imager 301 as illustrated in Figure 3.

The OCTA volume and OCT volume data 302 is first passed to an optional pre-processing processer 303. The pre-processing processer 303 first detects regions with OCT or OCTA signals above background noise. Background regions can be excluded in the later processing steps to speed up the processing time. Then landmarks are detected along each OCT/OCTA A-line (depth-direction). These landmarks may include peaks and valleys along the 1D A-line signal profile, and are often associated with retinal layer boundaries. For example, inner limiting membrane (ILM), junction of inner and outer photoreceptor segments (IS/OS), and retinal pigment epithelium (RPE) usually have stronger OCT intensities and appear as peak points along OCT A-lines. The locations or depths of these landmarks can be further refined by averaging over neighboring landmarks (across A-lines and across B-scans). Next, flattening is performed to align all A-scans to a chosen landmark in depth. This is a common step performed for retina segmentation and has been disclosed previously. *See, e.g.,* Mona K. Garvin et al. "Automated 3-D Intraretinal Layer Segmentation of Macular Spectral-Domain Optical Coherence Tomography Images," IEEE Trans Med Imaging, Sep 2009.

If the optional pre-processing processer 303 is not applied, the OCTA and OCT volume 302 are passed to a feature extraction processer 304. If the optional pre-processing processer 303 is applied, the pre-processed OCTA and OCT volume, along with outputs from the pre-processing processer 303 (for example, detected landmarks) are passed to the feature extraction processer 304. Feature-extraction processer 304 extracts features for each base unit. The base unit can be one single voxel or a localized region formed by a small number of voxels. These features include but are not limited to spatial location or depth of the current base unit; pre-processed OCT and OCTA intensities; features based on the nature of projection artifact to consider the projection of anterior true flow onto the posterior tissue layers; and information related to vessel caliber.

Feature extraction involving spatial location or depth of the current base unit can include, for example, distance to landmarks (measured in pixels or in microns). Such extraction may also include relative distance (RD) to landmarks, for example, the relative distance from the current base unit ($z_{current}$) to landmark A ($z_A$) can be computed by normalizing with the distance between landmark A and B ($z_B$). This can be given by the following relation:

$$RD_A(z) = |z_{current} - z_A| / |z_A - z_B|.$$

Feature extraction involving pre-processed OCT and OCTA intensity can include the OCT intensity of the current base unit and the OCTA intensity of the current base unit. Furthermore, derivatives (1st, 2nd, ...) of OCT intensity

in each x-, y-, z-direction from the current base unit and derivatives ($1^{st}$, $2^{nd}$, ...) of OCTA intensity in each x-, y-, z-direction from the current base unit can be included. Furthermore, intensities and derivatives neighboring base units can be used. The kernel size of the neighboring base units to be included as features can be fixed. For example, for a base unit of one single voxel, the surrounding 26 voxels in a $3\times3\times3$ kernel can be defined as neighbors. The kernel size can also be dynamically determined. For example, a bigger kernel size can be assigned to a voxel with a higher OCTA intensity.

Feature extraction based on the nature of the projection artifact to consider the projection of anterior true flow onto the posterior tissue layers can include depth-cumulative OC TA intensity along an A-line:

$$OCTA_{cum}(z) = \sum_0^z I_{OCTA}$$ , where depth is indexed from 0 between the anterior and the posterior. Such features can also include the maximum OCTA intensity along an A-line: $OCTA_{max}(z) = argmax[OCTA(z), z \in \{0,z\}]$. Such features may also include a corresponding OCT intensity at the same depth location where maximum OCTA intensity along A-line is detected. Yet another example of these features includes One-dimensional (1D) derivative of $OCTA_{max}(z)$.

Feature extraction may also include information related to vessel caliber. Such features include the distance to a closet base unit with half the OCTA intensity of the current base unit in the x-direction, the distance to the closet base unit with half the OCTA intensity of the current base unit in the y-direction, the distance to the closet base unit with half the OCTA intensity of the current base unit in the +z-direction, or the distance to the closet base unit with half the OCTA intensity of the current base unit in -z-direction. After the features are extracted, some of the features can be further combined to become a single feature.

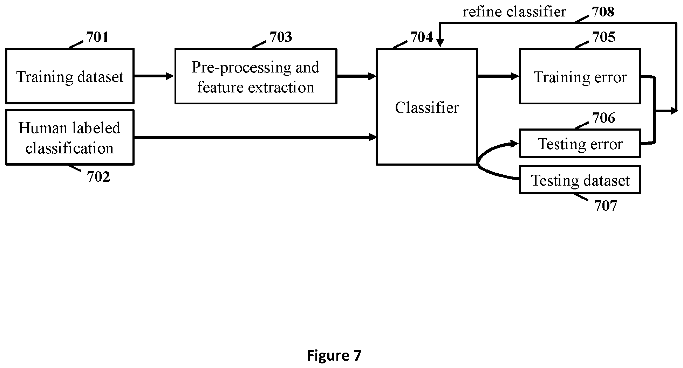

After the features have been extracted, the extracted features are passed to a classifier (block 305). The classifier is trained with a sufficiently large dataset where each OCTA voxels are manually labeled by human experts to indicate the presence of different types of artifacts, including projection artifacts. The details of the how the classifier can be trained is described in the Training classifier section and Figure 7. In some embodiments, the classifier can also be designed with observational criteria. The classifier then returns the probability or score of each base unit belonging to one of the classification categories. For example, three categories can be used: a purely true flow signal, a purely artifact signal, and a mixture of both true flow and artifact. In some embodiments, the classifier can return a hard classification which predicts which categories the base unit belongs to, without providing the probability.

Next, the probability volume or categorical results provided by the classifier is passed to a transform processer (block 306). Due to the complexity of the projection artifacts where mixtures of true and false signals occur frequently, the percentage of true signal in each base unit needs to be determined for successful artifacts reduction. The processer therefore transforms the probability or categorical results to the percentage of true signal in each base unit. The transform function can be a linear transformation determined empirically by phantom studies or by optimizing human retinal scan data to meet clinical understanding. For example,

$$Percentage_{true} = w_1 \cdot Prob_{true} + w_2 \cdot Prob_{mixed} + w_0,$$

where $Percentage_{true}$ is the percentage of true signal in the base unit, $Prob_{true}$ and $Prob_{mixed}$ is the probability of belonging to a purely true flow signal group and the probability of belonging to a mixed signal group, respectively. The parameters $w_0$, $w_1$, and $w_2$ are the linear weighting factors, which may be determined empirically.

Once the percentage is calculated for each base unit, the percentage value is assigned to every voxel in the base unit. Finally, the artifacts are reduced by multiplying the percentage with the pre-processed OCTA data ($OCTA_{pre}$) for each voxel (block 307)

$$OCTA_{post}(x,y,z) = OCTA_{pre}(x,y,z) \cdot Percentage_{true}(x,y,z),$$

and the post-processed artifacts-reduced OCTA volume ($OCTA_{post}$) is obtained. The artifacts-reduced OCTA data can then be utilized for display (block 308) including but not limited to 3D visualization with volume rendering, 2D visualization of en face projection images and B-scans. The artifacts-reduced OCTA data can also be used for further analysis (block 309) to calculate flow or vasculature-related quantitative parameters.

Figure 4 illustrates an exemplary image of the OCTA B-scan at the same location as Fig. 1 after projection artifacts are reduced. The arrows indicate a few locations where the projection artifacts are reduced after processing. Elongated inner retinal vessels which appear in the pre-processed B-scan (Figure 1) are shortened. This circular shape of vessels is more consistent with their physical dimensions. Projection artifacts at the IS/OS and RPE layers are also significantly reduced.

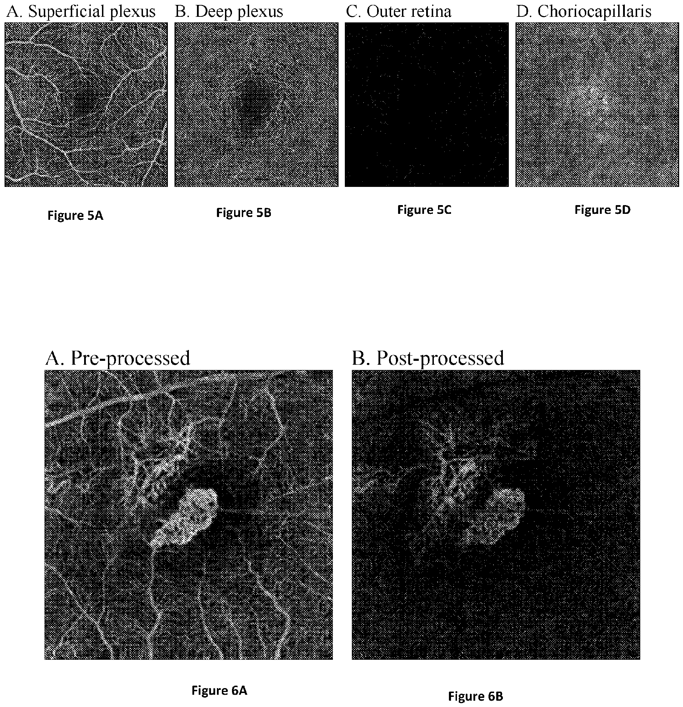

Figures 5A through 5D illustrate exemplary images of OCTA imaging of the same normal subject shown in Figures 2A through 2D after the projection artifacts are reduced. The four en face images include superficial capillary plexus (Figure 5A), deep capillary plexus (Figure 5B), outer retina (Figure 5C), and choriocapillaris (Figure 5D). Figures 5A through 5D show the post-processed en face images as compared to the pre-processed en face images in Figures 2A through 2D. The duplicated vascular networks are removed from the bottom layers, while the remaining networks are preserved and well-connected.

Figures 6A and 6B illustrates exemplary images of the outer retina of an AMD patient with CNV before (Figure 6A) and after (Figure 6B) projection artifacts are reduced in the OCTA volume. After processing, the projection artifacts are reduced and the CNV network is better visualized (Fig. 6B). The CNV boundaries are also easier to outline which allows more reliable patient follow-up to assess treatment outcome.

### *Training Classifier*

Figure 7 shows a block diagram illustrating the steps to train the classifier used for projection artifacts reduction. The classifier used in the projection artifacts reduction process, illustrated as block 305, can be pre-trained with a sufficiently large amount of data. Figure 7 is an exemplary flow diagram demonstrating the steps to train the classifier. First a training dataset with co-acquired OCT and OCTA volume data from subjects with varying ages, genders and retinal pathologies are collected by an OCT/OCTA imager in block 701. A-lines are randomly selected from the OCT/OCTA volume for normal subjects, and randomly selected within pathological areas in scans of pathological patients. Then human experts grade every base unit of these A-lines in block 702. Each base unit is labeled with a category. For example, the categories can include pure flow signal, pure projection artifacts signal, mixed signal, noise, and unknown signal. A subset of dataset is used as testing dataset in block 707 and not used during the training process.

The OCT and OCTA volume data goes through the pre-processing and feature extraction step in block 703 as described in the previous sections. The volume data, features and the human graded label are then passed to a classifier in block 704. The machine learning model, for example, can be based on logistic regression, ensemble models such as random forest, naive bayes, support vector machine, or combinations of different models. The training error is calculated during the training process in block 705. After the classifier is trained, the testing dataset (block 707) is inputted to the classifier and the testing error is calculated in block 706. The training error (block 705) and testing error (block 706) are then used to refine the classifier in block 708. During this step, the parameters and features in the classifier are refined to minimize while balancing the error from the training dataset and from the testing dataset.

The method described herein is applied to reduce projection artifacts in OCTA volume, but other artifacts such as noise and shadowing artifacts, can also be reduced through the same processing. The method can also be applied to detect artifacts in OCT volume, such as shadowing artifacts.

The invention is defined by the appended claims.

### Claims

1. A computer implemented method of reducing artifacts, comprising:

   obtaining optical coherence tomography OCT angiography OCTA data from an OCT/OCTA imager (301);
   preprocessing OCTA/OCT volume data to form preprocessed OCTA/OCT volume data;
   extracting features from the preprocessed OCTA/OCT volume data;
   classifying the OCTA/OCT volume data to provide a probability determination data with a classifier (305), the classifier being trained to return a probability of each base unit in the volume data to belong to one of a set of classification categories, the base unit being a single voxel or a localized region formed by a small number of voxels, the probability determination data reflecting the probability of each base unit being a member of a classification in the set of classification categories;
   determining a percentage data from the probability determination data, the percentage data reflecting a weighted sum of the probability determination data; and
   reducing artifacts in response to the percentage data by multiplying the preprocessed OCTA/OCT volume data by the percentage data.

2. The method of claim 1, wherein preprocessing OCTA/OCT volume data, comprises:

   detection of regions with the OCTA/OCT signals above background noise;
   excluding regions in the OCTA/OCT signals that are not above background noise;
   detecting landmarks along each OCTA/OCT A-line; and
   flattening to align all of A-scans with a chosen landmark.

3. The method of claim 1, wherein extracting features includes extracting features in each base unit of the OCTA/OCT data.

4. The method of claim 3, wherein the base unit is a single voxel.

5. The method of claim 3, wherein the base unit is a plurality of voxels.

6. The method of claim 1, wherein the set of classification categories includes a purely true flow signal, a purely artifact signal, or a mixture of both true flow and artifact signals.

7. The method of claim 1, wherein training the classifier comprises:

provism a training dataset;

providing a training dataset;
preprocessing the training dataset;
extracting features in the training dataset;
classifying the training dataset to obtain probability determination data;
comparing the probability determination data with human labeled probability data;
refining the trained classifier such that the probability determination data matches the human labeled probability data.

8. The method of claim 1, wherein determining a percentage data from the probability determination data includes a transform equation or matrix to transform the probability of the base unit belonging to each category in the set of classification categories to a single true flow signal percentage value in the base unit.

**Patentansprüche**

1. Computerimplementiertes Verfahren zum Reduzieren von Artefakten, das Folgendes beinhaltet:

Gewinnen von OCT-(Optical Coherence Tomography) -Angiographie-(OCTA)-Daten von einem OCT/OCTA-Bildgeber (301);
Vorverarbeiten von OCTA/OCT-Volumendaten, um vorverarbeitete OCTA/OCT-Volumendaten zu bilden;
Extrahieren von Merkmalen aus den vorverarbeiteten OCTA/OCT-Volumendaten;
Klassifizieren der OCTA/OCT-Volumendaten zum Bereitstellen von Wahrscheinlichkeitsbestimmungsdaten mit einem Klassifizierer (305), wobei der Klassifizierer trainiert wird, um eine Wahrscheinlichkeit zurückzugeben, dass jede Basiseinheit in den Volumendaten zu einer aus einem Satz von Klassifizierungskategorien gehört, wobei die Basiseinheit ein einzelnes Voxel oder eine durch eine geringe Anzahl von Voxeln gebildete lokalisierte Region ist, wobei die Wahrscheinlichkeitsbestimmungsdaten die Wahrscheinlichkeit widerspiegeln, dass jede Basiseinheit ein Mitglied einer Klassifikation in dem Satz von Klassifikationskategorien ist;
Bestimmen von Prozentdaten aus den Wahrscheinlichkeitsbestimmungsdaten, wobei die Prozentdaten eine gewichtete Summe der Wahrscheinlichkeitsbestimmungsdaten widerspiegeln; und
Reduzieren von Artefakten als Reaktion auf die Prozentdaten durch Multiplizieren der vorverarbeiteten OCTA/OCT-Volumendaten mit den Prozentdaten.

2. Verfahren nach Anspruch 1, wobei das Vorverarbeiten von OCTA/OCT-Volumendaten Folgendes beinhaltet:

Erkennen von Regionen mit den OCTA/OCT-Signalen über dem Hintergrundgeräuschpegel;
Ausschließen von Regionen in den OCTA/OCT-Signalen, die nicht über dem Hintergrundgeräuschpegel liegen;
Erkennen von Landmarken entlang jeder OCTA/OCT-A-Linie; und
Abflachen zum Ausrichten aller A-Scans mit einer gewählten Landmarke.

3. Verfahren nach Anspruch 1, wobei das Extrahieren von Merkmalen das Extrahieren von Merkmalen in jeder Basiseinheit der OCTA/OCT-Daten beinhaltet.

4. Verfahren nach Anspruch 3, wobei die Basiseinheit ein einzelnes Voxel ist.

5. Verfahren nach Anspruch 3, wobei die Basiseinheit eine Mehrzahl von Voxeln ist.

**6.** Verfahren nach Anspruch 1, wobei der Satz von Klassifikationskategorien ein rein wahres Flusssignal, ein reines Artefaktsignal oder eine Mischung aus sowohl wahren Fluss- als auch Artefaktsignalen umfasst.

**7.** Verfahren nach Anspruch 1, wobei das Training des Klassifizierers Folgendes beinhaltet:

Bereitstellen eines Trainingsdatensatzes;
Vorverarbeiten des Trainingsdatensatzes;
Extrahieren von Merkmalen in dem Trainingsdatensatz;
Klassifizieren des Trainingsdatensatzes, um Wahrscheinlichkeitsbestimmungsdaten zu erhalten;
Vergleichen der Wahrscheinlichkeitsbestimmungsdaten mit vom Menschen markierten Wahrscheinlichkeitsdaten;
Verfeinern des trainierten Klassifizierers, so dass die Wahrscheinlichkeitsbestimmungsdaten mit den vom Menschen markierten Wahrscheinlichkeitsdaten übereinstimmen.

**8.** Verfahren nach Anspruch 1, wobei das Bestimmen von Prozentdaten aus den Wahrscheinlichkeitsbestimmungsdaten eine Transformationsgleichung oder -matrix beinhaltet, um die Wahrscheinlichkeit, dass die Basiseinheit zu jeder Kategorie in dem Satz von Klassifizierungskategorien gehört, in einen einzelnen wahren Flusssignal-Prozentwert in der Basiseinheit zu transformieren.

**Revendications**

**1.** Un procédé mis en œuvre par ordinateur de réduction d'artefacts, comprenant :

l'obtention de données d'angiographie de tomographie par cohérence optique, OCT, OCTA, à partir d'un imageur OCT/OCTA (301),
le prétraitement de données de volume OCTA/OCT de façon à former des données de volume OCTA/OCT prétraitées,
l'extraction de caractéristiques des données de volume OCTA/OCT prétraitées,
le classement des données de volume OCTA/OCT de façon à fournir des données de détermination de probabilité avec un classifieur (305), le classifieur étant formé de façon à renvoyer une probabilité de chaque unité de base dans les données de volume d'appartenir à une catégorie d'un ensemble de catégories de classement, l'unité de base étant un voxel unique ou une zone localisée formée par un petit nombre de voxels, les données de détermination de probabilité reflétant la probabilité de chaque unité de base d'être un membre d'un classement dans l'ensemble de catégories de classement,
la détermination de données de pourcentage à partir des données de détermination de probabilité, les données de pourcentage reflétant une somme pondérée des données de détermination de probabilité, et
la réduction d'artefacts en réponse aux données de pourcentage par la multiplication des données de volume OCTA/OCT prétraitées par les données de pourcentage.

**2.** Le procédé selon la Revendication 1, où le prétraitement de données de volume OCTA/OCT, comprend :

la détection de zones avec les signaux OCTA/OCT au-dessus d'un bruit de fond,
l'exclusion de zones dans les signaux OCTA/OCT qui ne sont pas au-dessus d'un bruit de fond,
la détection de points de repère le long de chaque ligne A OCTA/OCT, et
un aplatissement destiné à aligner la totalité des balayages A avec un point de repère choisi.

**3.** Le procédé selon la Revendication 1, où l'extraction de caractéristiques comprend l'extraction de caractéristiques dans chaque unité de base des données OCTA/OCT.

**4.** Le procédé selon la Revendication 3, où l'unité de base est un voxel unique.

**5.** Le procédé selon la Revendication 3, où l'unité de base est une pluralité de voxels.

**6.** Le procédé selon la Revendication 1, où l'ensemble de catégories de classement comprend un signal purement de flux vrai, un signal purement d'artefact, ou un mélange à la fois de signaux d'artefact et de flux vrai.

**7.** Le procédé selon la Revendication 1, où la formation du classifieur comprend :

la fourniture d'un ensemble de données de formation,

le prétraitement de l'ensemble de données de formation,

l'extraction de caractéristiques dans l'ensemble de données de formation,

le classement de l'ensemble de données de formation de façon à obtenir des données de détermination de probabilité,

la comparaison des données de détermination de probabilité à des données de probabilité étiquetées par un humain,

l'affinage du classifieur formé de sorte que les données de détermination de probabilité correspondent aux données de probabilité étiquetées par un humain.

8. Le procédé selon la Revendication 1, où la détermination de données de pourcentage à partir des données de détermination de probabilité comprend une équation de transformée ou une matrice destinée à transformer la probabilité de l'unité de base appartenant à chaque catégorie dans l'ensemble de catégories de classement en une valeur de pourcentage de signal de flux vrai unique dans l'unité de base.

ILM

RPE

Figure 1

A. Superficial plexus    B. Deep plexus    C. Outer retina    D. Choriocapillaris

Figure 2A

Figure 2B

Figure 2C

Figure 2D

Figure 3

Figure 4

A. Superficial plexus    B. Deep plexus    C. Outer retina    D. Choriocapillaris

**Figure 5A**    **Figure 5B**    **Figure 5C**    **Figure 5D**

A. Pre-processed    B. Post-processed

**Figure 6A**    **Figure 6B**

701 Training dataset

703 Pre-processing and feature extraction

702 Human labeled classification

704 Classifier

refine classifier 708

705 Training error

706 Testing error

707 Testing dataset

**Figure 7**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 43670417
- US 10194866 B
- US 62297649 A
- US 2015062590 A1
- US 2015110348 A1
- US 8781214 B2, John Davis

**Non-patent literature cited in the description**

- **TALISA E. DE CARLO et al.** A review of optical coherence tomography angiography (OCTA). *Int J Ret Vit,* April 2015
- **RICHARD F. SPAIDE et al.** Image Artifacts in Optical Coherence Tomography Angiography. *Retina,* November 2015
- **YALI JIA et al.** Quantitative Optical Coherence Tomography Angiography of Choroidal Neovascularization in Age-Related Macular Degeneration. *Ophthalmology,* July 2014
- **ANQI ZHANG et al.** Minimizing Projection Artifacts for Accurate Presentation of Choroidal Neovascularization in OCT Micro-Angiography. *Biomed Opt Exp,* September 2015
- **MIAO ZHANG et al.** Projection-resolved optical coherence tomographic angiography. *Biomed Opt Exp,* March 2016
- **YALI JIA et al.** Split-spectrum amplitude-decorrelation angiography with optical coherence tomography. *Optics Express,* February 2012
- **MONA K. GARVIN et al.** Automated 3-D Intraretinal Layer Segmentation of Macular Spectral-Domain Optical Coherence Tomography Images. *IEEE Trans Med Imaging,* September 2009